# EUROPEAN PATENT SPECIFICATION

(11) **EP 2 694 013 B1**
(45) Date of publication and mention of the grant of the patent: **18.07.2018**
(21) Application number: 11849905.2
(22) Date of filing: 05.04.2011
(51) Int. Cl.: A61K 8/34, A61K 8/44, A61K 8/02, A61K 8/88, A61K 8/89, A61Q 1/06

(54) **Dual core lipstick**
Zweikern-Lippenstift
Stick à lèveres à double noyau

(43) Date of publication of application: 12.02.2014
(73) Proprietor: Avon Products, Inc., New York, NY 10017 (US)
(72) Inventor: DO, Thi N., West Orange, New Jersey 07052 (US); MCNAMARA, William E., Chester, New York 10918 (US); HALES, Kelly, Nyack, New York 10960 (US); TRAVKINA, Irina, River Edge, New Jersey 07661 (US)
(74) Representative: Dr. Weitzel & Partner
(86) International application number: PCT/US2011/031207
(87) International publication number: WO 2012/138326

(56) References cited:
- US-A1- 2007 243 151
- US-A1- 2008 107 695
- US-A1- 2008 274 354
- US-A1- 2009 280 076
- US-A1- 2009 280 077
- US-A1- 2010 150 855

## Description

### TECHNICAL FIELD

The present disclosure relates to the cosmetic, cosmeceutical, and personal care arts. In particular, a combination of components that results in a clear or translucent composition may be processed using existing manufacturing equipment under specific conditions. The processes of the instant disclosure leads to the formation of a clear or translucent composition obtained in an unexpected and highly beneficial manner.

### BACKGROUND

Cosmetic formulations or personal care products may be prepared in a solid, semi-solid, or gel composition. For example, compressed powder sticks, gel sticks, and wax sticks are traditionally used. These formulation types have advantages in certain situations, however, each also has disadvantages. Compressed powder sticks are known to be brittle and hard, and have a tendency to leave a cosmetically-unacceptable dust upon application. While gels can provide very good aesthetic characteristics, they may be unstable due to, for example, undesirable interactions between gelling agents which are typically used to solidify such sticks and the "active" ingredient (e.g., sunscreens and antiperspirant salts). Wax-based formulations can also yield cosmetically-unacceptable products due to its hardness, greasiness, and stickiness. Another disadvantage of wax-based stick formulations is that the wax results in an opaque formulation which interferes with a colorant's true color from being presented. In addition, tradition wax based formulations tend to be much harder, that is the compositions are not soft and often result in a draggy application.

Another problem in the art is that clear stick compositions tend to sweat one or more solvents under elevated temperature conditions. Additionally, such sticks melt under high temperature conditions and potentially become cloudy when cycled between freezing conditions and ambient temperature which is typically 25°C. Generally, these elevated temperatures and/ or freezing temperatures occur during storage and the transportation of the stick product.

Translucent compositions, mainly in the form of lip gloss or lip-stick, that are non-tacky, easily usable and have good storage stability have been disclosed in US 2009/0280076 A1.

It is therefore an object of the disclosure to provide a clear or translucent composition, that has sufficient strength to avoid solvent from seeping out from the composition at high temperatures, capable of cycling between various temperatures and maintaining clarity and that transmits a true colour. This is realised with a dual core lipstick as described herein.

### SUMMARY

It is one object of the disclosure to provide for a lipstick as follows: A lipstick comprising a translucent outer shell surrounding a colored inner core, wherein the translucent outer shell and the colored inner shell comprise: (a) N-lauroyl-L-glutamic acid dibutyl amide and N-2-ethylhexanoyl-L-glutamic acid dibutyl amide; (b) octyldodecanol in an amount of 15% to 30% by total weight of the composition; (c) polyamide resins comprising ethylenediamine hydrogenated dimer dilinoleate copolymer Bis-Di-C14-18 alkyl amide; (d) stearyl dimethicone; and (e) hydrogenated polyisobutene, wherein said composition has a dissolution temperature at or below about 115.°C; and wherein the colored inner shell comprises colorants having a surface treated with a fluoropolymer or a combination of fluoropolymers.

This embodiment relates to the clear or translucent composition in a stick format.

It is produced a clear or translucent stick with reproducible hardness. Without this higher stick strength, the sticks cannot be removed from the manufacturing moulds without damage.

In yet another object of the disclosure, the lipstick is a cosmetic, a cosmeceutical, or a personal care product.

In the lipstick a colored composition with colorants and the clear or translucent composition are combined. The colorants include traditional colorants at least some with surface treatment(s), goniochromatic colorants, multi-dimensional pigments, pearlescents, and holographic glitters.

In a further object of the disclosure, the unique combination of components or ingredients produces a clear or translucent composition that is processed using existing cosmetic manufacturing equipment.

Yet another object relates to a method for the manufacture of the clear or translucent stick with a high stick strength.

Also disclosed is a method of producing the lipstick composition that comprises mixing components (a), (b), (c), (d) and (e), thereby producing the clear or translucent composition where the mixture of (a), (b), (c), (d) and (e) has a dissolution temperature of about 115 °C or less and is effected in about 10 to about 20 minutes depending upon batch size and conditions. It should be noted that once dissolution has been achieved the composition may be cooled to a pour temperature less than 115 °C.

These and other objects of the present disclosure will become apparent to those skilled in the art after a reading of the following detailed description, including the illustrative embodiments and examples.

### DETAILED DESCRIPTION

The present disclosure is directed to a lipstick as defined in the claims.

One of the problems in the art of stick compositions is the lack of composition strength which is required during manufacture and production. Without enough or sufficient stick strength, the composition is oftentimes does not eject from the mould or is damaged when ejected or removed from a mould. The composition of the present invention employs two amino acid - based gelatinizing agents increased to a significant amount greater than what is found in the art, the polyamide resin known as Ethylenediamine Hydrogenated Dimer Dilinoleate Copolymer Bis-Di-C14-18 Alkyl Amide, and one or more alkyl dimethicone materials to form the clear or translucent composition of the disclosure. The amino acid-based gelling agents of the invention have the structure according to formula (I): 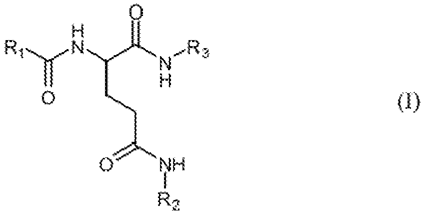 and are N-lauroyl-L-glutamic acid dibutyl amide and N-2-ethylhexanoyl-L-glutamic acid dibutylamide.

As the skilled artisan understands, dissolution occurs when all of the ingredients or components of a composition completely dissolve. However, in order to achieve complete dissolution of the prior art compositions comprising amino acid - based gelling agents, the temperature must be elevated at or above 130 °C followed by a lengthy gelling time of at least 15 hours in order to cool the composition sufficiently in order to gel at a temperature of about 23 °C to about 25 °C. Although the amount of amino acid - based gelling agent(s) used in the prior art compositions is generally extremely small (1:100 gellants to oil ratio), the amino acid - based gellants must still be processed under extreme conditions for prolonged periods of time as stated above in order to achieve a gel. In particular, a temperature ranging from about 130 °C to over about 200 °C is typically required for those amino acid - based gelatinizing agents, as discussed above and found in the prior art, to go into solution. Because of the extremely high temperature required for dissolution, prior art compositions which require these gelatinizing agents were limited to a select group of oils or polymers which could withstand the extremely high temperatures.

Another disadvantage of using these gelatinizing agents is that the time for them to gel typically takes a very long time. For example, for the gels in the prior art to set, the required time and temperature would be about 15 hours at 25 °C. Therefore, processing these gelatinizing agents in the art utilizes a lot of energy due to the high dissolution temperature, reduces the amount of output in view of the lengthy time required to obtain the product, limits the oils or polymers that could be used, and increases the total manufacturing costs.

Whereas, the particular combination of ingredients or components of the composition of the present disclosure allows for dissolution at much lower temperatures and thereby requires a shorter amount of time to obtain the disclosed clear or translucent gel composition having improved properties over the art. As previously stated, the combination of the amino acid - based gellants, the Ethylenediamine Hydrogenated Dimer Dilinoleate Copolymer Bis-Di-C14-18 Alkyl Amide, fatty alcohol, alkyl dimethicone, and oils or polymers, achieves a dissolution at or below 115°C in a matter of minutes to a few hours depending upon the overall batch size. Additionally, the composition of the present invention is capable of setting in a matter of minutes as compared to about 15 hours as is the case for gels of the prior art. The composition of the present invention may be moulded, ejected and inserted in a package in the span of 3 to 10 minutes depending upon choice of equipment and set conditions.

The amino acid - based gelatinizing agents of the inventive composition comprise a mixture of at least two amino acid - based gelatinizing agents, three or more amino acid - based gelatinizing agents may also be useful. Non-limiting examples include any of the known gelatinizing agents, N-acetyl glutamic acid dilauryl amide, N-caproyl glutamic acid dibutyl amide, N-lauryl glutamic acid dihexyl amide, N-lauroyl-glutamic acid dihexyl amide, and N-acetyl amino acid amides. In a specific embodiment, the mixture of amino acid - based gelatinizing agents includes N-lauroyl-L-glutamic acid dibutyl amide and N-2-ethylhexanoyl-L-glutamic acid dibutyl amide. If there are two amino acid - based gelatinizing agents, then they are in a weight ratio ranging between 1:3 or 3:1, where a preferred ratio is 2:3. However, in another embodiment where there are more than two amino acid -based gelatinizing agents, the weight ratio is, for example, 1:1:1. A particularly preferred ratio is 2:3 dibutyl ethylhexanoyl glutamide : dibutyl lauroyl glutamide.

Furthermore, the mixture of or total amount of amino acid - based gelatinizing agents is more than the aforementioned prior art gellants. Another embodiment of the disclosure is directed to a composition comprising amino acid - based gellants having a weight percent of about 1.5 % to about 6.0 % of the total composition, about 2.25 % to about 3.0 %, or about 3.5 % to about 5.0 % of the total composition, in contrast to the typically less stable stick compositions in the art which comprise amino acid - based gelatinizing agents less than 1 % by weight of the total composition. Moreover, less stable stick compositions in the art typically employ one or two amino acid - based gellant(s), and such compositions do not include polyamide resin, non-ionic fatty alcohol or alkyl dimethicone or derivatives thereof. The synergistic combination of the amino acid - based gellants, Ethylenediamine Hydrogenated Dimer Dilinoleate Copolymer Bis-Di-C14-18 Alkyl Amide and the alkyl dimethicone allows for the production of a more rigid, cosmetic sticks that are capable of withstanding extreme environmental conditions whilst maintaining their integrity. In the prior art, very high temperatures were necessary to effect dissolution of the amino acid - based gellants. However, dimethicone and polyamide resin materials are not processed at such high temps. Furthermore, the polyamide resin and dimethicone help stabilize the stick in two ways: first, the polyamide resin makes the stick more rigid; and second, the combination of polyamide resin and dimethicone prevent the stick from "sweating." When compositions are exposed to freezing conditions or high temperatures, such as during storage or transportation, the compositions will tend to become hazy and may sweat or exude solvent when returning to ambient conditions from either of the extreme temperatures listed. However, due to the unique combination of ingredients disclosed herein, sweating and haze is not problematic.

The lipstick comprises in the shells of the composition at least one fatty alcohol which can dissolve the amino acid - based gelatinizing agents or gellants at or below a temperature of 115 °C. Therefore, the lipstick comprises octyldodecanol in an amount of 15% to 30% by total weight of the composition. One or more further non-ionic unsaturated fatty alcohols useful for dissolution includes but is not limited to a non-ionic mono- or poly- unsaturated fatty alcohol. Non-limiting examples of useful fatty alcohols of the disclosure include oleyl alcohols, octyldodecanols, 2-butyloctanals, 2-hexyldecanols, and 2-undecylpentadecanols. A particular embodiment is directed to Octyldodecanol. The long chain, unsaturated fatty alcohol of the inventive composition is present in an amount from about 0.1% to about 45 % by weight of the total composition. Other embodiments are directed to an amount of about 15 % to about 30 %, and further, about 16 % to about 25.5 % by weight of the total composition.

Selection of the fatty alcohol is dependant upon its ability to dissolve the amino acid-based gellants at or below 115 °C. In one embodiment, the useful unsaturated fatty alcohols are nonvolatile. Nonvolatile is defined herein as those fatty alcohols having at 1.0 atmosphere, a boiling point of at least about 200 °C, at least about 205 °C, and at least about 210 °C. Some fatty alcohols of the instant disclosure have, for example, one double bond (monounsaturated). Their general formula is: CH₃(CH₂)ₓCH=CH(CH₂)_{y}-CH₂OH.

Suitable fatty alcohols also include unsaturated monohydric straight chain fatty alcohols, saturated branched chain fatty alcohols, saturated C8-C12 straight chain fatty alcohols, and mixtures thereof. The unsaturated straight chain fatty alcohols will typically have one degree of unsaturation. Di- and tri- unsaturated alkenyl chains may be present at low levels, less than about 5 % by total weight of the unsaturated straight chain fatty alcohol, less than about 2%, or less than about 1 %. In certain embodiments, the unsaturated straight chain fatty alcohols have an aliphatic chain length ranging from C12 - C22, from C12 - C18, or from C16 - C18, such as, for example, oleyl alcohol and palmitoleic alcohol. Non-limiting examples of non-ionic unsaturated fatty alcohols include those identified in Table 1:

**TABLE 1**

| Common Name | Chemical Name | Molecular Structure | # Carbons |
|---|---|---|---|
| | | | |
| palmitoleyl alcohol | (cis-9-hexadecen-1-ol) | CH₃(CH₃)₅CH=CH(CH₂)₈OH | 16 |
| | | | |
| elaidyl alcohol | (9E-octadecen-1-ol) | CH₃(CH₂)₇CH=CH(CH₂)₆OH | 18 |
| | | | |
| oleyl alcohol | (cis-9-octadecen-1-ol) | CH₃(CH₂)₇CH-CH(CH₂)₈OH | 18 |
| | | | |
| linoleyl alcohol | (9Z, 12Z-octadecadien-1-ol) | polyunsaturated | 18 |
| | | | |
| elaidolinoleyl alcohol | (9E, 12E-octadecadien-1-ol) | polyunsaturated | 18 |
| | | | |
| linolenyl alcohol | (9Z, 12Z, 15Z-octadecatrien-1-ol) | polyunsaturated | 18 |
| | | | |
| elaidolinolenyl alcohol | (9E, 12E, 15-E-octadecatrien-1-ol) | polyunsaturated | 18 |
| | | | |
| ricinoleyl alcohol | (12-hydroxy-9-octadecen-1-ol) | polyunsaturated | 18 |
| | | | |
| ricinoleyl alcohol | (12-hydroxy-9-octadecen-1-ol) | CH₃(CH₂)₅CH(OH)CH₂CH=C H(CH₂)₈OH unsaturated, diol | 18 |
| | | | |
| erucyl alcohol | (cis-13-docosen-1-ol) | CH₃(CH₂)₇CH=CH(CH₂)₁₂OH | 22 |

In an additional embodiment, the polyamide resin provides a substantial level of structural integrity when the present invention takes the semi-solid or solid form. Polyamide resins are high molecular weight polymers which feature amide linkages along the molecular chain. These polymers contain monomers of amides joined by peptide bonds, they can occur both naturally and artificially, such polymers are made through step growth polymerization or solid phase synthesis, in some cases, examples of polyamide resins are nylons and aramids. Due to their extreme durability and strength polyamide resins are typically utilized in textiles, plastics and various automotive applications. In the composition of the present invention the polyamide resin also provides a degree of gloss or shine to the composition and adhesion to the target substrate.

While the polyamide resin Ethylenediamine Hydrogenated Dimer Dilinoleate Copolymer Bis-Di-C14-18 Alkyl Amide is comprised in the lipstick of the invention, other poyamide resins may also be comprised. Further additional polyamide resins and many suitable polymers are disclosed in the CTFA Handbook, 12th Ed. 2008. These include, without limitation, Polyamide-1, Polyamide-2, Polyamide-3, Ethylenediamine/Dimer Tallate Copolymer Bis-Hydrogenated Tallow Amide, Ethylenediamine / Stearyl Dimer Dilinoleate Copolymer, Ethylenediamine / Stearyl Dimer Tallate Copolymer.

A particular embodiment of the present disclosure is directed to a polyamide resin, or a combination of compatible polyamide resins, in an amount ranging from about 0.1 % to about 30 % by weight of the total composition, about 10 % to about 25 % by weight, and about 15 % to about 20 %. A particularly preferred polyamide resin is Ethylenediamine Hydrogenated Dimer Dilinoleate Copolymer Bis-Di-C14-18 Alkyl Amide.

The alkyl dimethicone component provides a thermally stable stick composition of the disclosure. In addition to improving the structure strength of the disclosed composition thereby easing removal of the composition from a mould, alkyl dimethicone and the polyamide resin provides structural integrity and helps prevent stick compositions from "sweating" in elevated temperatures, for example, during storage or product transport. Clear stick compositions in the art tend to 'sweat' or 'weep' one or more solvents at elevated temperatures. These high temperatures are generally between about 130 °C and above 200 °C. In order to overcome these problems in the art, an alkyl dimethicone material (which relates to one or more alkyl dimethicones) may be included in the clear or translucent composition of the disclosure.

As is understood in the art, alkyl dimethicones can be either silicone- or oil- soluble, depending on the ratio of x, y and z in the below formula.

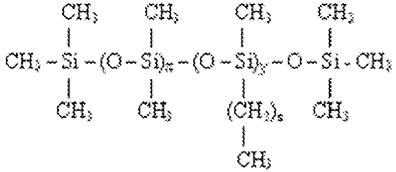

As the value of x and y increases, the alkyl dimethicone become harder and more soluble in silicone oil. As the z value increases, the alkyl dimethicone will be more soluble in oils, such as vegetable, mineral, and ester, which also raises the melting temperature of the waxes. As the value of x increases and the value of z decreases, the more soluble the alkyl dimethicone becomes in silicone. However, as the values of y and z increase, the alkyl dimethicone becomes more oil soluble.

As is well known by skilled artisans in the formulations art, varying the ratio of x, y and z determines whether alkyl dimethicone is a liquid, a soft paste or a hard wax. Generally, using a higher number of alkyl groups, for example 22 carbons, the product will be a paste or hard wax, depending on the ratio of x to y. A higher value of x lowers the melting point of the product. While, an olefin with 18 carbon groups, the alkyl dimethicone can be a liquid to hard wax, depending on the ratio of x to y. Alkyl groups with 16 carbon groups or lower are normally liquids to soft pastes. There has been found an improvement in SPF values when alkyl dimethicones are added to sunscreens. Therefore, one of ordinary skill in the art who will consider the factors related to the desired results, will determine the exact ratios of x, y, and z. The ratios are adjusted to provide structure or to maintain the desired effect. Factors, which may be taken into account, include the use and desired form of the final product.

A particular embodiment of the present disclosure is directed to an alkyl dimethicone, or a combination of compatible alkyl dimethicones, in an amount ranging from about 0.1 % to about 30 % by weight of the total composition, about 10 % to about 25 % by weight, and about 15 % to about 20 %, The alkyl dimethicone useful in the instant disclosure has an alkyl in the range of about 18 % to 65 % by weight of the entire alkyl dimethicone, while the dimethicone is in the corresponding range of about 35 % to about 82 % by weight of the alkyl dimethicone. In another embodiment, alkyl dimethicones containing from 1 to 18 carbons, such as, for example an alkyl dimethicone of stearyl dimethicone C18, are most useful. Additional non-limiting examples of alkyl dimethicones of the instant disclosure include behenyl dimethicone, C-32 alkyl dimethicone, isopropyl phenyl dimethicone, cerotyl dimethicone, hydroxypropyl diemthicone behenate, cetyl/hexacosyl dimethicone, and the D and J series of Silwax® alkyl dimethicones and Multi Domain™ silicones (Siltech LLC; Dacula, GA).

According to the present invention, as alkyl dimethicone, at least stearyl dimethicone is comprised in the lipstick.

The polyamide resin employed to provide additional structural integrity to the stick composition of the present is Ethylenediamine Hydrogenated Dimer Dilinoleate Copolymer Bis-Di-C14-18 Alkyl Amide. This polyamide resin may be used in the composition of the present invention in the amount of about 1 % to about 35 %, or about 5 % to 25 % by weight of the total composition.

In yet a further embodiment, the lipstick as described may comprise in the shells in addition a combination of multiple oils, multiple polymers or both, i.e., a combination of one or more oils and/or polymers. The oil or polymer of the disclosure includes, but is not limited to, non-polar oils, polar oils, liquid polymers, solution polymers, or combinations thereof. The oils used for the clear or translucent composition of the present disclosure that are suitable for purposes of this composition include those that sufficiently allow the dissolution of the gelatinizing agents upon heating and thereby resulting in the formation of a gel when cooled. Non-limiting examples include mineral oils, isohexadecane, jojoba oils, C10 - C30 cholesterol/ Lanoesterol esters, e.g. Super Sterol Liquid (Croda Inc.; Edison, NJ), mink oil, cacao oil, coconut oil, palm seed oil, camellia oil, sesame oil, castor oil, olive oil, aloe extract, and silicone oil.

More specifically, illustrative ester oils include esters such as tridecyl trimellitate, , triisostearyl citrate, diisostearate maleate, diisostearyl fumarate, and the like. Liquid polymers or solution polymers useful in the present disclosure include, but are not limited to, polybutene, hydrogenated polyisobutene, triisostearyl polyglyceryl-3-dimer dilinoleate, polyglycerol diisostearate , and, such as. The oil or polymer component of the composition disclosed herein may be in an amount ranging from about 20 % to about 70 % by weight of the total composition, more particularly, ranging from about 32.5 % to about 47.5 % of the total composition, or about 42 % to about 65 % of the total composition.

Without being bound by theory, in order to obtain a clear or translucent composition that has the benefit of a relatively fast dissolution and gelling time, as well as low dissolution and gelling temperature, that has relatively increased stick strength, and reduced sweating properties, the fatty alcohol, such as Octyldodecanol, that is compatible with the amino acid - based gelatinizing agents, in particular, N-lauryol-L-glutamic acid dibutylamide and N-2-ethyl-hexanoyl-L-glutanic acid dibutylamide in a weight ratio ranging between 1:3 or 3:1, or more specifically, 2:3, provides complete dissolution at or below 115 °C and in a matter of minutes.

In yet another embodiment, the clear or translucent composition comprises additional components. The clear or translucent composition may additionally include an antioxidant or mixture thereof to protect the composition from thermal degradation. In a particular embodiment, one or more antioxidants may be used in an amount ranging from about 0.01 % to about 0.5 % by weight of the total composition. One antioxidant useful in the clear or translucent composition of the disclosure is a butylated hydroxyl toluene (BHT). However, any antioxidant compatible with the other components of the inventive composition may be used, including but not limited to potassium sulfite, sodium bisulfite, butylated hydroxyanisole, and the like. The antioxidant may be in an effective amount to protect the composition from degrading, such as for example, between about 0.01 % to about 0.5 % by weight of the total composition, about 0.06 % to about 0.5 %, and about 0.3 % to about 0.5 % by weight. Examples 1 and 2 provide exemplary clear or translucent formulations.

Traditional stick compositions employ high melting point waxes, such as paraffin wax, beeswax, carnauba wax, ozokerite wax, microcrystalline, and polyethylene wax are used in order to provide the composition with structure. Such waxes have high melting points and create a matrix where the structure is comprised of randomly orientated crystals resulting in an opaque appearance. As a result, these waxes interfere with the colorants and the resulting visualized color is not accurate. Therefore, in another embodiment of the present disclosure, the clear or translucent composition combined with either traditional colorants and/ or goniochromatic colorants delivers a "true color" with a vibrancy only observed thus far in clear liquid media.

In the lipstick, the compositions further comprise one or more coloring agents. It is within the skill in the art to select coloring agents and combinations of coloring agents to produce a desired color or effect. Suitable traditional coloring agents, including pigments, lakes, and dyes, are well known in the art and are disclosed in the C.T.F.A. Cosmetic Ingredient Handbook, First Edition, 1988, the contents or which are hereby incorporated by reference. Organic pigments include, for example, FD&C dyes, D&C dyes, including D&C Red, Nos. 2, 5, 6, 7, 10, 11, 12, 13, 30 and 34, D&C Yellow No. 5, Blue No. 1, Violet No. 2. Exemplary inorganic pigments include, but are not limited to, metal oxides and metal hydroxides such as magnesium oxide, magnesium hydroxide, calcium oxide, calcium hydroxides, aluminum oxide, aluminum hydroxide, iron oxides (α-Fe₂O₃, y-Fe₂O₃, Fe₃O₄, FeO), red iron oxide, yellow iron oxide, black iron oxide, iron hydroxides, titanium dioxide, titanium lower oxides, zirconium oxides, chromium oxides, chromium hydroxides, manganese oxides, cobalt oxides, cerium oxides, nickel oxides and zinc oxides and composite oxides and composite hydroxides such as iron titanate, cobalt titanate and cobalt aluminate. Other suitable coloring agents include ultramarine blue (*i.e*., sodium aluminum silicate containing sulfur), Prussian blue, manganese violet, bismuth oxychloride, talc, mica, sericite, magnesium carbonate, calcium carbonate, magnesium silicate, aluminum magnesium silicate, silica, titanated mica, iron oxide titanated mica, bismuth oxychloride, and the like. The coloring agents may be surface modified, with, for example, fluoropolymers, to adjust one or more characteristics of the coloring agent as described in, for example, U.S. Patent Nos. 6,471,950, 5,482,547, and 4,832,944, the contents of which are hereby incorporated by reference. Fluoropolymers are incorporated into the present disclosure as a coating on pigment particles that at least partially covers the surface of the pigment particles. Suitable pearling pigments include without limitation bismuth oxychloride, guanine and titanium composite materials containing, as a titanium component, titanium dioxide, titanium lower oxides or titanium oxynitride, as disclosed in U.S. Patent No. 5,340,569, the contents of which are hereby incorporated by reference.

In one embodiment of the disclosure, traditional colorants are employed in an amount ranging from about 0.01 % to about 30 % by weight of the total composition. The combination of any such colorant with the herein disclosed clear or translucent composition delivers a true color rarely seen in traditional solid formulations. In yet another embodiment of the disclosure, the goniochromatic colorants useful in the inventive compositions described herein is in an amount ranging from about 0.1 % to about 15 % by weight of the total composition. The goniochromatic colorants, also known as color travel pigments, include platelet-shaped base substrates coated with alternating layers of high or low refractive index materials. Goniochromatic colorants are pigments that exhibit different colors depending on the viewing angle.

Illustrative colorants include Reflecks™ MultiDimensions (BASF Corp.; Florham Park, NJ) pigments that can shift across the entire visual spectrum to provide multiple color effects through its borosilicate pigment technology, and SpectraFX which is a powdered flake additive available in a variety of sizes from very small to extra large (Alsa Corp.; Vernon, CA). Reflecks™ MultiDimensions pigments are composed of a calcium sodium borosilicate base coated with silica, titanium dioxide, and tin oxide. In a particular embodiment of the disclosure, goniochromatic colorants were added to the clear or translucent composition to deliver unique color effects. These goniochromatic colorants range in particle size from about 1 micrometer to about 100 micrometers, or more specifically, from about 40 micrometers to about 80 micrometers, as exemplified with SpectraFX pigments and Reflecks™ pigments, respectively.

A further embodiment is directed to the amount of goniochromatic colorant useful in the clear or translucent composition of the disclosure, which ranges from about 0.1 % to about 15 % by weight of the total composition, while in a specific embodiment, the amount of colorant ranges from about 0.5 % to about 5 % by weight of the total composition. The particle size and amount of colorant used in the composition determines the final optical effect of the composition. As is understood by the person of ordinary skill in the art, a weight percent load of 0.5 % of the Copper/Patina SpectraFX pigment results in translucency as well as a perceived color shift from copper/ patina to green depending on the viewing angle. Whereas, at higher colorant loadings, the translucency of the composition is diminished due to the fact that less light may travel through the composition. However, there is still a perceived color shift depending upon the viewing angle.

Similarly, in yet a further embodiment, combined with the clear or translucent composition, pearlescent type colorants may be used to deliver better or more true color effects than seen in a traditional opaque, wax-based stick composition. Another embodiment is directed to the clear or translucent composition with holographic glitters that produce unique effects. These glitters include those that are holographically embossed, vacuum metalized polyester flakes, such as those available from Spectratek Technologies, Inc. (Los Angeles, CA). In the same way that the visual effects of the SpectraFX pigments varies depending on the load amount, the same holds true for the holographic glitters. When added at a load of less than about 2 % by weight of the total composition, the inventive composition combined with the holographic glitters produce a translucent appearance, and the glitters look like they are suspended within the composition, such that different colors are produced by the different glitter particles. However, at higher colorant loads, the translucency is diminished.

In yet a further embodiment, the clear or translucent composition is combined with a mixture of traditional colorants and goniochromatic colorants, i.e., not a dual core style as described above. When the inventive composition is used in such a manner, the typical amount of traditional colorant ranges from about 0.1 % to about 25 % by weight of the total composition and the goniochromatic colorant ranges from about 0.1 % to about 15 % of the total composition.

Another embodiment is directed to a method of making the clear or translucent composition as described herein. One embodiment which utilizes the traditionally used machinery, comprises simultaneously mixing all of the components, including: the ingredients (a)-(e) of the shells as earlier described, at a temperature of about 115 °C or lower sufficient to dissolve the components. Typically dissolution occurs within minutes to hours depend on batch size. Once dissolution is achieved, the resulting mixture may be cooled to a lower pour temperature. The gel or set temperature denotes when the molten composition begins to change from a free flowing liquid to a gel, semi-solid or solid composition. The gel or set temperature useful in the instant disclosure ranges from about 25 °C to about 80 °C, from about 65 °C to about 80°C or in the most useful embodiment, around room temperature or about 25 °C. Once again, the time necessary for the clear or translucent composition to gel is on the scale of minutes and not hours. Example 2 is directed to an example of a method of producing the clear or translucent composition of the instant disclosure.

In yet a further embodiment of the disclosure, the method is directed to mixing components (a) and (b) first, heating the mixture of (a) and (b) to a temperature of at most about 115 °C or lower thus dissolving the mixture. Subsequently, components (c), (d) and (e) are added either simultaneously or subsequently of each other, and dissolved at a similar temperature, *i.e*., no more than about 115 °C. The resultant mixture of components (a), (b), (c), (d) and (e) is then cooled to to form a solid, semi-solid or viscous gel composition.

Another embodiment of the disclosure is directed to the addition of optional ingredients that contribute other benefits to the inventive composition. Non-limiting active or functional ingredients may include colorants, pigments, ultraviolet filters, moisturizing agents, fragrances, insecticides, pesticides, pharmaceutical agents, and other active or functional ingredients known in the cosmetic or pharmaceutical arts. These additional ingredients may be mixed simultaneously with the components of (a), (b), (c), (d) and (e), or with components (c), (d) and (e), or subsequent to the mixture of any or all of the components of (a), (b), (c), (d) or (e). Regardless, the additional ingredients must be compatible with the parameters of the inventive composition, *i.e*., for example, dissolution at a temperature at or below about 115 °C. It should be noted that optional ingredients may be added at lower temperatures as dictated by physical or thermal limitations of said materials or the form of the desired end product. A further embodiment is directed to any of the aforementioned methods comprising an additional step of casting the molten mixture or viscous gel into a mould. It should be noted that once dissolution has been achieved, the resultant composition may be cooled from 115 °C to some lower temperature depending upon desired filling conditions and form choice of the final product. Typically, such compositions are filled or moulded on automated equipment wherein the time scale from pour to mould to eject to package insertion ranges anywhere from 2 to 10 minutes. In addition, said automated equipment mould temperatures may range from about -10°C to about 40°C depending upon the desired product form and performance attributes associated with such.

The present invention is directed to a dual core style configuration. For example, the inventive clear or translucent composition may form an outer shell and the traditional lipstick forms an inner core such that the outer shell surrounds the inner core which is visible through the clear or translucent outer shell forming concentric circles. Similarly, the colored inventive composition may form the inner core, such that even though the clear or translucent outer shell surrounds the inner core, the colors from the inner core are visible. infinite color combinations are possible, in addition special color effects are possible by incorporating goniochromatic pigments in the outer, clear shell such that they are complimentary to the inner core coloration, or in certain embodiments, the colorant is in the inner core. The loading of colorant in the different concentric portions is dictated by the desired end visual effect as applied or as stored. Yet another embodiment of the instant disclosure relates to a light source being employed to project light into the clear gel thus illuminating the gel and providing additional visual effects. Non-limiting examples of light sources may be incandescent, infrared, laser, fluorescent, and natural. In embodiment, the invention relates to a packaged lip care product comprising a container for holding a lip composition, a cap on the container to limit exposure of the composition to the air when not in use, and a mechanism associated with the container for dispensing the composition from the container. The container comprises a tube charged with a composition according to the invention and a light source, such as an LED, in the body of the container which is capable of illuminating the composition when the composition is exposed, for example by removing the cap on the container or by pressing an actuator or switch.

In another embodiment, the composition of the disclosure may also include any additive usually employed in the field envisaged such as antioxidants, perfumes, essential oils, stabilizers, cosmetic active substances, moisturizers, vitamins, essential fatty acids, lipophilic sunscreens, liposoluble polymers, and especially hydrocarbon polymers such as polyalkylenes and polyamides for improving smoothness or spreadability, water and oil resistance, transfer resistance, or other cosmetic or cosmeceutical properties desired by one of skill in the art. Non-limiting examples of optionally added ingredients include: emollients, thickening agents, for example, opacifying agents, clays, or organoclays, silicas, cellulose derivatives, plasticizers, gels, fatty acids, fats, powders, oils, preservatives, solvents, surfactants; hectorites; synthetic polymers such as an acrylic polymer or an associative polymer of the polyurethane type; spreading agents; dispersants; preservatives, antifoaming agents; wetting agents; ultraviolet-screening agents; perfumes; fillers and bulking agents; binders; cosmetic or pharmaceutical active agents; moisturizers; feel enhancers including but not limited to powders and oils; vitamins and derivatives thereof; and biological materials and derivatives thereof. If the softness and elasticity of the composition are to be increased still further, it is also possible to add a plasticizer, which is commonly added for cosmetic materials. Suitable materials may include both low-molecular weight and high-molecular weight plasticizers, which are optionally used, solubilized, or dissolved in a co-solvent.

Suspending and thickening agents typically include silica gels, gums, clays, fumed silica, fatty acid soaps, and various hydrocarbon gels, and other ingredients that when incorporated into the formulation remain on the surface of keratinous tissues. Non-limiting examples of ingredients, such as emollients, that may preferably be used in the compositions of the disclosure include glycerine, propylene glycol, cyclomethicone, dimethicone, and emollients and other similar ingredients disclosed in the International Cosmetic Dictionary and Handbook Vols. 1 and 2. Eds. Wenninger, J.A. and G.N. McEwen, Cosmetic, Toiletry, and Fragrance Association, Washington DC, 2000, which is hereby incorporated by reference.

A pigment that is not necessarily one of the goniochromatic pigments described above, should be understood to mean inorganic or organic, white or colored particles. Coloring agents that may be used in the practice of the disclosure may include pigments, lakes, and dyes which are well known in the art and are disclosed in the Cosmetic Ingredient Handbook, First Edition, J.M. Nikitakis, et al., Cosmetic, Toiletry, and Fragrance Association, Washington DC, 1988, the contents or which are hereby incorporated by reference. Depending on the application for the composition, pigments may be added to provide color or no color.

Other embodiments may be directed to the inventive compositions with fillers, mother-of-pearl, and the like, to modify the texture of the composition and the matte or glossy appearance. Fillers should be understood to mean lamellar or nonlamellar, inorganic or synthetic, colorless or white particles. Mother-of pearl should be understood to mean iridescent particles produced especially by certain mollusks in their shell or else synthesized. Pearling agents that may be used in the practice of the disclosure include mica, iron oxides, titanium dioxide and any other pearling agent known in the cosmetic arts. Non-limiting examples of fillers and microspheres used either alone or in combination, for example, the pressed powder composition prototypes include: talc, corn starch nylon powder, polymethyl methacrylate, polytetraflourothylene, zinc stearate, boron nitride, calcium silicate, and the like.

Compounds commonly used in the cosmetic arts for preventing or reducing fungal, bacterial, or microorganismal growth are also added to the composition of the disclosure. By including these compounds, the shelf life of the composition is lengthened. These anti-fungal and anti-microorganisms include but are not limited to methyl paraben, butyl paraben, sodium dehydroacetate, etc. The amounts of these anti-fungal or anti-microorganism ingredients are in an amount effective to reduce growth without negatively affecting the components of the inventive composition or the desired effects.

The person skilled in the art will of course take care to choose the optional additional compounds and/or their quantities in such a way that the advantageous properties of the composition according to the disclosure are not, or are substantially not, impaired by the envisaged addition(s). In embodiments where these materials are added to the formulations of the disclosure to enhance the spreadability and the emollience of the product, however, it is preferred that the above materials be present in low enough concentrations for the formulation to retain its desired properties. These ingredients may be selected variously by the person skilled in the art in order to prepare a composition which has the desired properties, for example, composition strength, true color optical effect, and dissolution temperature of about 115 °C or less on the order of within minutes versus hours. The choice of additional ingredients and their concentrations may also be adjusted to vary the desired properties. In one embodiment, a fragrance may be added for superficial purposes, such as for example, to make the inventive composition more appealing to the consumer.

A further embodiment of the disclosure includes lip stick compositions including reflectors, absorbers, or filters of ultraviolet rays to provide a suntan lotion or sunblock.

The packaging of the inventive composition into, for example, a kit or article of manufacture, and application device for any embodiment of the disclosure is chosen and manufactured by persons skilled in the art on the basis of their general knowledge, and adapted according to the nature of the composition to be packaged. Moreover, the type of device to be used may be in particular linked to the consistency of the composition, in particular to its viscosity; it may also depend on the nature of the constituents present in the composition, such as the presence of volatile compounds. The kit or article of manufacture may include, but is not limited to, the inventive composition, a device for the application of the inventive composition, instructions for the use and application of the inventive composition, a listing of ingredients and/or warnings, and the like. The article of manufacture is one that presents the stick composition as herein described.

### EXAMPLE

The following example further describes and demonstrates embodiments within the scope of the present disclosure. The example is given solely for the purpose of illustration and is not to be constructed as limitations of the present disclosure, as many variations thereof are possible without departing from the spirit and scope of the disclosure. The following exemplary composition is given by way of illustration only and in no way intended to be limiting to the scope of disclosure.

### EXAMPLE 1

### CLEAR OR TRANSLUCENT STICK COMPOSITION

All of the ingredients identified in Table 2 were mixed using a rotor-stator high shear equipment at a speed of between about 500 and 10,000 rpm at a temperature of about 115 °C for about 5 to about 15 minutes or until a clear solution was obtained. Although a "regular" mixer using low shear could be used, the process would take longer. The clear solution was poured into a mould to form a clear or translucent stick composition.

**TABLE 2**

| Ingredients | Weight Percent (%) |
|---|---|
| EB-21 | 6.00 |
| GP-1 | 9.00 |
| Octyldodecanol | 85.00 |

### EXAMPLE 2

### CLEAR OR TRANSLUCENT STICK COMPOSITION

All of the ingredients identified in Table 3 were mixed using a rotor-stator high shear equipment at a speed of between about 500 and 10,000 rpm at a temperature of about 115 °C for about 5 to about 15 minutes or until a clear solution was obtained. The clear solution was poured into a mould to form a clear or translucent stick composition.

**TABLE 3**

| Ingredients | Weight Percent (%) |
|---|---|
| Dibutyl Ethylhexanoyl Glutamide | 2.00 |
| Dibutyl Lauroyl Glutamide | 3.00 |
| Octyldodecanol | 28.00 |
| hydrogenated polyisobutene | 38.95 |
| Ethylenediamine Hydrogenated Dimer Dilinoleate Copolymer Bis-Di-C14-18 Alkyl Amide | 15.80 |
| stearyl dimethicone | 5.00 |
| BHT | 0.25 |
| ethylhexyl methoxycinamate | 7.00 |

### EXAMPLE 3

### METHOD OF PREPARING A CLEAR OR TRANSLUCENT COMPOSITION

In order to obtain a clear or translucent composition, the components were combined such that the dissolution temperature and gel temperature were lower than what is commonly found or expected based on the characteristics of the individual components. The time for the components to dissolve and form a gel was also much shorter, on the scale of minutes to hours (batch size from 100 grams to 2 kilograms).

The mixture of Table 2 was heated to a temperature of 115 °C for about 5 to about 15 minutes, or until a clear solution is obtained The resulting gel concentrate was combined with the preheated mixture of oils or polymers, as well as additional optional ingredients, such as an antioxidant, a sunscreen, and a fragrance as indicated in Table 4 below.

**TABLE 4**

| Ingredient | Weight Percent (%) |
|---|---|
| Gel Concentrate of Table 2 | 20 - 30 |
| Alkyl dimethicone | 5 - 25 |
| Polyamide Resin | 5 - 25 |
| Oil | 40 - 70 |
| Antioxidant | 0.01 - 0.5 |
| Sunscreen | 5 - 7 |
| Fragrance | 0 - 0.5 |

The resulting molten formulation was cast into a mould and cooled at a temperature of about -10 °C to about 40 °C for about 3 to 10 minutes to form a clear or translucent stick composition of the disclosure.

The composition listed in table 5 is a non limiting example of a colored composition, that may be used on its own or as part of a multiphase product as in the case of a center core lipstick. Wherein the composition listed in table 5 serves as the inner core and the composition of table 3 serves as the outer shell thus creating a formula within a formula resulting in the inner core being visible through the outer shell. In this instance it is preferable to use colorants that are treated with fluoropolymers to inhibit color migration from the inner core formula into the clear outer shell.

**TABLE 5**

| Ingredient | Weight Percent (%) |
|---|---|
| HYDROGENATED POLYISOBUTENE | 11.70 |
| DIISOSTEARYL MALATE | 5.00 |
| DIMER DILINOLEYL DIMER DILINOLEATE | 5.00 |
| STEARYL DIMETHICONE (MP: 28.5 - 31.0) | 3.60 |
| ETHYLHEXYL-METHOXYCINNAMATE | 5.00 |
| TOCOPHERYL ACETATE-SYN | 0.70 |
| OCTYLDODECANOL | 17.00 |
| DIBUTYL ETHYLHEXANOYL GLUTAMIDE | 1.50 |
| DIBUTYL LAUROYL GLUTAMIDE | 2.20 |

**TABLE 5**

| Ingredient | Weight Percent (%) |
|---|---|
| Ethylenediamine Hydrogenated Dimer Dilinoleate Copolymer Bis-Di-C14-18 Alkyl Amide | 11.00 |
| HYDROGENATED POLYISOBUTENE/GELLANTS/BHT | 13.00 |
| TIT.DIOXIDE/TITANATE TRTD | 1.95 |
| D&C RED NO 7-99 2%/FLUORO-SILANE TRTD | 5.85 |
| IRON OXIDES-RED ISOPROP. TIT. TRIISOST.(ITT) TRTD. | 5.20 |
| CLOISONNE BLUE FLAMBE #650Z | 4.50 |
| TIMICA EXTRA BRIGHT 1500 | 4.80 |
| SILICA 2-20 MICRO SPHERE MSS 500/3 | 2.00 |

## Claims

1. A lipstick comprising a translucent outer shell surrounding a colored inner core, wherein the translucent outer shell and the colored inner shell comprise: (a) N-lauroyl-L-glutamic acid dibutyl amide and N-2-ethylhexanoyl-L-glutamic acid dibutyl amide; (b) octyldodecanol in an amount of 15% to 30% by total weight of the composition; (c) polyamide resins comprising ethylenediamine hydrogenated dimer dilinoleate copolymer Bis-Di-C14-18 alkyl amide; (d) stearyl dimethicone; and (e) hydrogenated polyisobutene, wherein said composition has a dissolution temperature at or below about 115.°C; and
wherein the colored inner shell comprises colorants having a surface treated with a fluoropolymer or a combination of fluoropolymers.

2. The lipstick of claim 1, wherein the amino acid-based gelatinizing agents are present in a weight ratio ranging from 1:3 to 3:1.

3. The lipstick of claim 1, further comprising at least one antioxidant.

4. The lipstick of claim 1, wherein said colorant is a pigment, a dye, a goniochromatic colorant, an aluminum salt, a pearlescent, a glitter or a combination thereof.

5. The composition of claim 4, wherein the colorant is present in an amount ranging from 0.5 weight % to 5.0 weight % of the total composition.

## Patentansprüche

1. Lippenstift, umfassend einen durchscheinenden äußeren Mantel, der einen farbigen inneren Kern umgibt, wobei der durchscheinende äußere Mantel und der farbige innere Kern folgendes umfassen: (a) N-Lauroyl-L-glutaminsäuredibutylamid und N-2-Ethylhexanoyl-L-glutaminsäuredibutylamid; (b) Octyldodecanol in einer Menge von 15% bis 30%, bezogen auf das Gesamtgewicht der Zusammensetzung; (c) Polyamidharze, umfassend Ethylenediamine/Hydrogenated Dimer Dilinoleate Copolymer bis-di-C14-18-Alkyl Amide; (d) Stearyldimethicon und
(e) hydriertes Polyisobuten, wobei die Zusammensetzung eine Lösetemperatur von etwa 115 °C oder weniger aufweist, und
wobei der farbige innere Kern Färbemittel mit einer Oberfläche aufweist, die mit einem Fluorpolymer oder einer Kombination von Fluorpolymeren behandelt ist.

2. Lippenstift nach Anspruch 1, wobei die Geliermittel auf Aminosäurebasis in einem Gewichtsverhältnis im Bereich von 1:3 bis 3:1 vorliegen.

3. Lippenstift nach Anspruch 1, ferner umfassend wenigstens ein Antioxidans.

4. Lippenstift nach Anspruch 1, wobei das Färbemittel ein Pigment, ein Farbstoff, ein goniochromatisches Färbemittel, ein Aluminiumsalz, Perlglanz, Glitzer oder eine Kombination davon ist.

5. Zusammensetzung nach Anspruch 4, wobei das Färbemittel in einer Menge im Bereich von 0,5 Gew.-% bis 5.0 Gew.-% der Gesamtzusammensetzung vorliegt.

## Revendications

1. Fard à lèvres comprenant une coque externe translucide entourant un noyau interne coloré, dans lequel la coque externe translucide et la coque interne colorée comprennent : (a) du dibutyl amide d'acide N-lauroyl-L-glutamique et du dibutyl amide d'acide N-2-éthylhexanoyl-L-glutamique ; (b) de l'octyldodécanol dans une quantité de 15 % à 30 % en poids total de la composition ; (c) des résines de polyamide comprenant du bis-di-alkyle (en C14 à 18) amide de copolymère de dilinoléate de dimère hydrogéné d'éthylènediamine ; (d) de la stéaryle diméthicone ; et (e) du polyisobutène hydrogéné, dans lequel ladite composition a une température de dissolution d'environ 115 °C ou moins ; et dans lequel la coque interne colorée comprend des colorants ayant une surface traitée avec un fluoropolymère ou une combinaison de fluoropolymères.

2. Fard à lèvres selon la revendication 1, dans lequel les gélifiants à base d'acide aminé sont présents dans un rapport en poids allant de 1 : 3 à 3 : 1.

3. Fard à lèvres selon la revendication 1, comprenant en outre au moins un antioxydant.

4. Fard à lèvres selon la revendication 1, dans lequel ledit colorant est un pigment, une teinture, un colorant goniochromatique, un sel d'aluminium, un agent perlé, une substance scintillante ou une de leur combinaison.

5. Composition selon la revendication 4, dans laquelle le colorant est présent dans une quantité allant de 0,5 % en poids à 5,0 % en poids de la composition totale.
